# EUROPÄISCHE PATENTSCHRIFT

(11) **EP 2 836 246 B1**
(45) Veröffentlichungstag und Bekanntmachung des Hinweises auf die Patenterteilung: **26.07.2017**
(21) Anmeldenummer: 13717714.3
(22) Anmeldetag: 11.04.2013
(51) Int. Cl.: A61M 1/36, G01M 3/16

(54) **VORRICHTUNG UND VERFAHREN ZUR ÜBERWACHUNG EINES GEFÄSSZUGANGS EINES PATIENTEN MIT EINEM GEWEBTEN FEUCHTESENSOR MIT KONTROLLABSCHNITT**
APPARATUS AND METHOD FOR MONITORING VASCULAR ACCESS OF A PATIENT COMPRISING A WOVEN MOISTURE SENSOR WITH A CONTROL PORTION
DISPOSITIF ET PROCÉDÉ DE SURVEILLANCE D'UN ACCÈS VASCULAIRE D'UN PATIENT AU MOYEN D'UN CAPTEUR D'HUMIDITÉ TISSÉ À SECTION DE CONTRÔLE

(30) Priorität: 11.04.2012 DE 102012007082; 11.04.2012 US 201261622677 P
(43) Veröffentlichungstag der Anmeldung: 18.02.2015
(73) Patentinhaber: Fresenius Medical Care Deutschland GmbH, 61352 Bad Homburg (DE)
(72) Erfinder: HEPPE, John, 66606 St. Wendel (DE)
(74) Vertreter: Oppermann, Frank
(86) Internationale Anmeldenummer: PCT/EP2013/001058
(87) Internationale Veröffentlichungsnummer: WO 2013/152855

(56) Entgegenhaltungen:
- EP-A1- 2 322 709
- WO-A1-2004/004615
- WO-A1-2006/008866
- WO-A1-2011/116943
- WO-A2-2009/075592
- JP-A- S54 133 196

## Beschreibung

### Gebiet der Erfindung

Die Erfindung betrifft das Gebiet der Überwachung des Gefäßzugangs eines Patienten gegen Blutverlust in die Umgebung mittels eines gewebten Feuchtigkeitssensors, insbesondere bei der extrakorporalen Blutbehandlung, und einer Auswertevorrichtung zur Detektion von Feuchte an dem Gefäßzugang des Patienten.

### Stand der Technik

Es sind verschiedene Arten von Blutbehandlungsvorrichtungen bekannt. Zu den bekannten Blutbehandlungsvorrichtungen zählen beispielsweise die Vorrichtungen zur Hämodialyse, Hämofiltration und Hämodiafiltration. Während der extrakorporalen Blutbehandlung strömt das Blut von einem arteriellen Gefäßzugang des Patienten durch eine arterielle Kanüle und durch einer arterielle Leitung des extrakorporalen Blutkreislaufs in eine Blutbehandlungseinheit, z.B. einen Dialysator, und nach Durchfließen der Blutbehandlungseinheit durch eine venöse Leitung des extrakorporalen Blutkreislaufs und durch eine venöse Kanüle zurück in den venösen Gefäßzugang des Patienten. Die Überwachung des Patienten während einer extrakorporalen Blutbehandlung gegen Blutverlust in die Umgebung ist eine ständige Herausforderung für das Personal einer Dialyseklinik. Besonders gefürchtet ist ein unfallmäßiger Freifluss des Bluts in die Umgebung aufgrund einer Diskonnektion oder Dislokation der rückführenden venösen Kanüle aus dem venösen Gefäßzugang. Aus dem Stand der Technik sind deshalb verschiedene Verfahren und Vorrichtungen zur Überwachung des Gefäßzugangs eines Patienten während einer Blutbehandlungssitzung gegen Blutverlust in die Umgebung bekannt. Beispielsweise sind Feuchtigkeitssensoren bekannt, die an der Punktionsstelle des Gefäßzugangs aufgeklebt werden können. Diese bekannten Feuchtigkeitssensoren beruhen auf dem Prinzip, dass das elektrisch leitfähige Blut den elektrischen Widerstand zwischen zwei Elektroden in den Feuchtigkeitssensoren verringert und der elektrische Widerstand durch eine Auswerteeinheit überwacht wird.

In der Schrift WO 2010/091852 A1 der Anmelderin Fresenius Medical Care Deutschland GmbH wird ein Feuchtigkeitssensor zur Überwachung eines Gefäßzugangs eines Patienten beschrieben, bei dem die Leiterbahnen und ein hochohmiger Abschlusswiderstand nachträglich auf einem Vlies aufgebracht, z.B. als leitfähige Druckfarbe oder Druckpaste aufgedruckt werden. Der aufgedruckte Abschlusswiderstand dient der Überprüfung der Funktion der Leiterbahnen bei trockenem Zustand des Feuchtigkeitssensors. Nachteil eines aufgedruckten Abschlusswiderstands ist der hohe Aufwand für das positionsgenaue Aufdrucken des Abschlusswiderstands, verbunden mit hohen Produktionskosten. Ein weiterer Nachteil ist die Gefahr lokaler Brüche oder Mikrorisse der aufgedruckten Paste bei mechanischer Belastung des Feuchtigkeitssensors, wodurch der elektrische Widerstand während der Anwendung des Sensors signifikant ansteigen kann.

In der Schrift WO 2011/116943 A1 der Anmelderin Fresenius Medical Care Deutschland GmbH wird ein durch Weben hergestellter Feuchtigkeitssensor zur Überwachung eines Gefäßzugangs eines Patienten beschrieben. Bei diesem Feuchtigkeitssensor werden die Leiterbahnen durch elektrisch leitfähige Kettfäden und Schussfäden in einem partiell mehrlagigen Gewebe realisiert. Elektrisch leitfähige Fäden haben üblicherweise den Zweck, den elektrischen Strom in Geweben mit möglichst geringem elektrischem Widerstand zu leiten. Zu diesem Zweck enthalten die bekannten Fäden beispielsweise Silber. Ein solcher Feuchtigkeitssensor ist vorgesehen zur Verwendung mit einem in einer Anschlussklemme integrierten externen Abschlusswiderstand, nämlich einem SMD (surface-mounted device), zur Überprüfung der Funktion der Leiterbahnen bei trockenem Zustand des Feuchtigkeitssensors. Der durch Weben hergestellte Feuchtigkeitssensor ist auf den externen Abschlusswiderstand angewiesen, weil die Integration eines definierten Abschlusswiderstands mit der erforderlichen hohen Reproduzierbarkeit, z.B. der eines SMD, in einen solchen Feuchtigkeitssensor aufwendig wäre und die beträchtlichen Kostenvorteile des Herstellprozesses durch Weben zum Teil zu Nichte machen würde. Ein solcher Feuchtigkeitssensor weist vier Anschlusskontakte auf, wobei zwei Anschlusskontakte für die Kontaktierung des SMD in der Anschlussklemme vorgesehen werden müssen. In der Internationalen Patentanmeldung PCT/EP2011/003044 der Anmelderin Fresenius Medical Care Deutschland GmbH wird eine solche Anschlussklemme für einen Feuchtigkeitssensor zur Überwachung eines Gefäßzugangs eines Patienten mit zwei Anschlusskontakten für die Leiterbahnen und zwei zusätzlichen Anschlusskontakten für den in der Anschlussklemme integrierten Abschlusswiderstand beschrieben. Nachteil dieser Anschlussklemme sind die Mehrkosten für die zwei Anschlusskontakte des integrierten Abschlusswiderstands. In der am Anmeldetag der vorliegenden Patentanmeldung noch unveröffentlichten Deutschen Patentanmeldung der Anmelderin Fresenius Medical Care Deutschland GmbH mit der Anmeldenummer DE 10 2011 113839.4 wird eine weitere Anschlussklemme für einen auf die Haut eines Patienten aufzulegenden Feuchtigkeitssensor zur Überwachung eines Gefäßzugangs des Patienten beschrieben.

Nachteil eines Anschlusskabels oder einer Anschlussklemme mit integriertem Abschlusswiderstand für einen Feuchtigkeitssensor ohne integrierten Abschlusswiderstand ist der erhöhte Fertigungsaufwand verbunden mit erhöhten Fertigungskosten.

In der am Anmeldetag der vorliegenden Patentanmeldung noch unveröffentlichten Deutschen Patentanmeldung der Anmelderin Fresenius Medical Care Deutschland GmbH mit der Anmeldenummer DE 10 2011 113838.6 wird ein Webverfahren zur Herstellung einer Vielzahl von Feuchtigkeitssensoren für die Überwachung eines Patientenzugangs beschrieben. Des Weiteren relevant sind die Schriften
- WO 2010/091852 A1,
- WO 2011/116943 A1,
   und die Anmeldungen
- DE 10 2011 113838.6 und
- DE 10 2011 113839.4 und
- PCT/EP2011/003044

Aus der JP 54-133196 A ist ein Feuchtigkeitssensor für Hochtemperatur-Rohrleitungssysteme bekannt, der eine elektrisch leitende Struktur mit gewobenen elektrisch leitfähigen Drähten aufweist, die an ihren Enden mit Elektroden elektrisch verbunden sind. Die WO 2009/075592 A2 beschreibt einen Feuchtigkeitssensor, der zur Detektion von Feuchtigkeit leitfähige Elemente aufweist, die auf oder in einem textilen Material vorgesehen sind. Die Drähte oder Leiterbahnen der beiden Feuchtigkeitssensoren selbst sind aber nicht derart ausgebildet, dass sie eine Überprüfung derselben erlauben.

### Aufgaben der vorliegenden Erfindung

Ein gattungsgemäßer gewebter Feuchtigkeitssensor weist mindestens eine durch Weben hergestellte Leiterbahn in einem mehrlagigen Gewebe auf, wobei die mindestens eine Leiterbahn aus leitfähigen Schussfäden und leitfähigen Kettfäden in dem ansonsten aus elektrisch nicht leitfähigen Fäden bestehenden Gewebe gebildet wird und die leitfähigen Schussfäden und leitfähigen Kettfäden an ausgewählten Kontaktpunkten elektrisch leitfähig miteinander verwebt sind. Eine Aufgabe der vorliegenden Erfindung ist es, einen gattungsgemäßen gewebten Feuchtigkeitssensor vorteilhaft weiterzubilden und Nachteile der aus dem Stand der Technik bekannten gewebten Feuchtigkeitssensoren zu überwinden.

Eine weitere Aufgabe der vorliegenden Erfindung ist es, einen gattungsgemäßen durch Weben hergestellten Feuchtigkeitssensor anzugeben, bei dem die Funktion der Leiterbahnen im trockene Zustand überprüft werden kann, ohne das dazu ein externer Abschlusswiderstand erforderlich ist.

Eine weitere Aufgabe der vorliegenden Erfindung ist es, einen gattungsgemäßen durch Weben hergestellten Feuchtigkeitssensor mit integriertem Abschlusswiederstand anzugeben, bei dem der Fertigungsaufwand gering ist.

Eine weitere Aufgabe der vorliegenden Erfindung ist es, einen gattungsgemäßen gewebten Feuchtigkeitssensor anzugeben, bei dem ein definierter Abschlusswiderstand im Gewebe integriert ist und der Ohm'sche Widerstand des Abschlusswiderstands verlässlich innerhalb vorgegebener Toleranzen reproduzierbar ist.

Eine weitere Aufgabe der vorliegenden Erfindung ist es, die Herstellkosten für eine Auswertevorrichtung zur Überwachung eines Gefäßzugangs eines Patienten mit einem gattungsgemäßen gewebten Feuchtigkeitssensor zu senken.

Eine weitere Aufgabe der vorliegenden Erfindung ist es, einen gattungsgemäßen gewebten Feuchtigkeitssensor anzugeben, der robust ist gegenüber mechanischen Belastungen.

### Die vorliegende Erfindung

Die Lösung dieser Aufgaben erfolgt erfindungsgemäß mit den Merkmalen der unabhängigen Patentansprüche 1 und 6. Vorteilhafte Ausführungsformen sind Gegenstände der Unteransprüche.

Es liegen im Stand der Technik bislang kaum Erfahrungen mit hochohmigen Fäden in elektrisch leitfähigen Geweben vor. Untersuchungen der Anmelderin haben jedoch gezeigt, dass eine ausreichende Reproduzierbarkeit eines allein durch Weben hergestellten hochohmigen Abschlusswiderstands gelingen kann, wenn zum Einen der Faden des Abschlusswiderstands mit einer hochohmigen Polymer-Boschichtung veredelt wird und zum Anderen die wirksame Länge des hochohmigen Abschlusswiderstands im Gewebe durch lokale leitfähige Kontaktpunkte mit kreuzenden leitfähigen Schussfäden und/oder kreuzenden leitfähigen Kettfäden des Feuchtigkeitssensor genau festgelegt und begrenzt wird.

Als besonders vorteilhafte hochohmige Polymer-Beschichtung hat sich eine sogenannte Carbon-Nanotube-Beschichtung herausgestellt, bei der die Reproduzierbarkeit besonders verlässlich ist. Fäden mit einer solchen Carbon-Nanotube-Beschichtung werden beispielsweise in der Schrift EP 2 322 709 A1 beschrieben.

Nach der Lehre der Erfindung werden die Aufgaben gelöst durch einen gewebten gattungsgemäßen Feuchtigkeitssensor mit mindestens einer durch Weben hergestellten Leiterbahn, die einen durch Weben hergestellten erfindungsgemäßen Kontrollabschnitt der Leiterbahn aufweist. Der Kontrollabschnitt der Leiterbahn besteht aus einem speziellen Abschnitt eines elektrisch leitfähigen Fadens.

Die vorliegende Erfindung sieht vor, dass der spezifische elektrische Widerstand des Kontrollabschnitts der Leiterbahn im Wesentlichen dem spezifischen elektrischen Widerstand der Fäden der Leiterbahn entspricht und dass der Kontrollabschnitt nach der Funktionsüberprüfung der Leiterbahn zerstörend von der Leiterbahn abgetrennt wird, so dass die Leiterbahn in mindesten zwei Elektroden aufgeteilt wird, wodurch der Feuchtigkeitssensor erst durch den Schritt des Abtrennens des Kontrollabschnitts für die Feuchtigkeitsmessung sensitiv wird. Bei dem erfindungsgemäßen Feuchtigkeitssensor kann ein Fehler in der Leiterbahn, beispielsweise eine Unterbrechung aufgrund eines Leiterbahnbruchs oder ein Webfehler erkannt werden, wenn der trockene Feuchtigkeitssensor an der Auswertevorrichtung angeschlossen ist, eine bekannte elektrische Prüfspannung angelegt wird und der gemessene Ohm'sche Widerstand zwischen den Anschlusskontakten des Feuchtigkeitssensors einen vorgegebenen ersten Widerstands-Grenzwert überschreitet (Leiterbahnbruch) oder einen zweiten Widerstands-Grenzwert unterschreitet (Kurzschluss) oder, wenn der elektrische Strom gemessen wird, der gemessene elektrische Strom einen vorgegebenen ersten Strom-Grenzwert unterschreitet (Leiterbahnbruch) oder einen zweiten Strom-Grenzwert überschreitet (Kurzschluss). Vorteil des erfindungsgemäßen Feuchtigkeitssensors ist, dass die Herstellung des Feuchtigkeitssensors besonders einfach und kostengünstig ist. Dafür muss allerdings in Kauf genommen werden, dass eine Funktionsüberprüfung der Leiterbahn nach dem Abtrennen des Kontrollabschnitts nicht mehr möglich ist. Untersuchungen haben aber gezeigt, dass aufgrund der im praktischen Einsatz am Patienten zu erwartenden mechanischen Beanspruchungen des Feuchtigkeitssensors die Leiterbahn nicht beschädigt werden kann.

Die vorliegende Erfindung sieht vor, dass der spezifische elektrische Widerstand des Kontrollabschnitts der Leiterbahn wesentlich größer ist als der spezifische elektrische Widerstand der Fäden der Leiterbahn und der Kontrollabschnitt als hochohmiger Abschlusswiderstand ausgeführt wird. Bevorzugt liegt der spezifische elektrische Widerstand des Kontrollabschnitts der Leiterbahn zwischen 10³ Ohm/cm und 10⁶ Ohm/cm. Besonders bevorzugt beträgt der spezifische elektrische Widerstand des Kontrollabschnitts 10⁴ Ohm/cm und 10⁵ Ohm/cm. Der Kontrollabschnitt der Leiterbahn wird aus einem Faden mit einer Carbon-Nanotube-Beschichtung hergestellt. Der hochohmige Faden kann fertigungsbedingt die gesamte Gewebebahn durchlaufen, z.B. als Kettfaden in Webrichtung oder als Schussfaden quer zur Webrichtung. Die Länge des als Abschlusswiderstand wirkenden hochohmigen Fadens im Gewebe wird erfindungsgemäß durch lokale leitfähige Verknüpfungen mit kreuzenden leitfähigen Schussfäden und/oder kreuzenden leitfähigen Kettfäden festgelegt. Dadurch wirkt nur ein genau definierter Abschnitt des hochohmigen Fadens als Abschlusswiderstand. Der absolute Betrag des wirksamen hochohmigen Abschlusswiderstands lässt sich erfindungsgemäß allein durch Weben innerhalb vorgegebener Toleranzen festlegen.

### Ausführungsbeispiele der vorliegenden Erfindung

Im Folgenden werden Ausführungsbeispiele des erfindungsgemäßen Feuchtigkeitssensors unter Bezugnahme auf die Figuren näher erläutert. Anhand der in den Figuren dargestellten Ausführungsbeispiele werden weitere Einzelheiten und Vorteile der Erfindung näher beschrieben. Die Bezugszeichen in den Figuren haben jeweils durchgängig in allen Figuren die gleiche Bedeutung.

Es zeigen:
- **Figur 1:**: schematische Darstellung eines gewebten Feuchtigkeitssensors entsprechend einem ersten Ausführungsbeispiel
- **Figur 2:**: schematische Darstellung eines gewebten Feuchtigkeitssensors entsprechend einem zweiten Ausführungsbeispiel
- **Figur 3:**: schematische Darstellung eines gewebten Feuchtigkeitssensors entsprechend einem dritten Ausführungsbeispiel

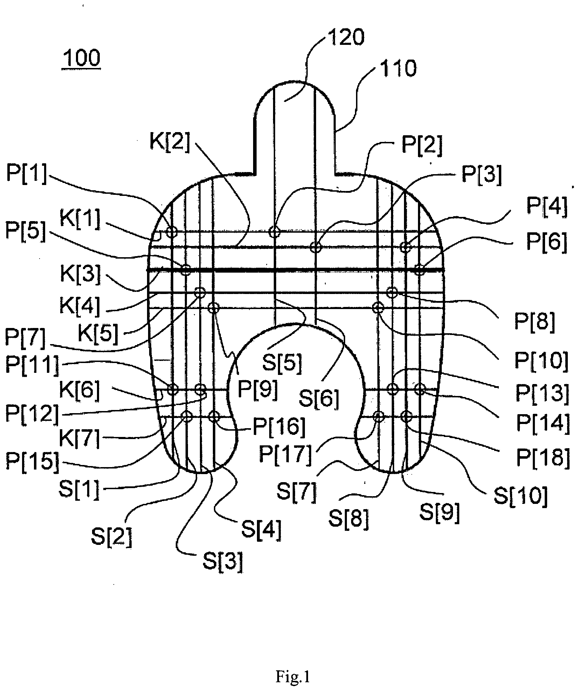

**Figur 1** zeigt in vereinfachter schematischer Darstellung einen erfindungsgemäßen gewebten Feuchtigkeitssensor 100, der durch die Außenkontur 110 begrenzt ist und eine Anschlusslasche 120 für die elektrische Kontaktierung des Feuchtigkeitssensors mit einer Anschlussklemme (nicht dargestellt) aufweist. Der im vorliegenden Ausführungsbeispiel gezeigte Feuchtigkeitssensor ist eine Weiterentwicklung des in der Schrift WO 2011/116943 A1 gezeigten Feuchtigkeitssensors. In dem gewebten Feuchtigkeitssensor 100 sind elektrische leitfähige Schussfäden S[1] bis S[10] und elektrische leitfähige Kettfäden K[1] bis K[7] in einem mehrlagigen Gewebe eingewebt. Die elektrisch leitfähigen Kettfäden K[1] bis K[7] sind mit den elektrisch leitfähigen Schussfäden S[1] bis S[10] ausschließlich an den ausgewählten Kontaktpunkten P[1] bis P[18] elektrisch leitend miteinander verwebt und ansonsten im mehrlagigen Gewebe gegeneinander isoliert. Die Schussfäden S[5] und S[6] verlaufen durch die Anschlusslasche 120. Aufgrund der Kontaktpunkte P[1] bis P[18] entsteht eine geschlossene Leiterbahn zwischen dem Schussfäden S[5] in der Anschlusslasche 120 und dem Schussfaden S[6] in der Anschlusslasche 120. Der Verlauf der Leiterbahn wird durch die spezielle Lage der Kontaktpunkte P[1] bis P[18] festgelegt. Die geschlossene Leiterbahn besteht im vorliegenden Ausführungsbeispiel der Figur 1 aus den nachfolgend genannten, leitend miteinander verbundenen Fadenabschnitten:
- Teilabschnitt im Schussfaden S[5] von der Anschlusslasche 120 bis zum Kontaktpunkt P[2],
- vom Kontaktpunkt P[2] bis zum Kontaktpunkt P[1],
- vom Kontaktpunkt P[1] bis zum Kontaktpunkt P[11],
- vom Kontaktpunkt P[11] bis zum Kontaktpunkt P[12],
- vom Kontaktpunkt P[12] bis zum Kontaktpunkt P[7],
- vom Kontaktpunkt P[7] bis zum Kontaktpunkt P[8],
- vom Kontaktpunkt P[8] bis zum Kontaktpunkt P[13],
- vom Kontaktpunkt P[13] bis zum Kontaktpunkt P[14],
- vom Kontaktpunkt P[14] bis zum Kontaktpunkt P[6],
- vom Kontaktpunkt P[6] bis zum Kontaktpunkt P[5],
- vom Kontaktpunkt P[5] bis zum Kontaktpunkt P[15],
- vom Kontaktpunkt P[15] bis zum Kontaktpunkt P[16],
- vom Kontaktpunkt P[16] bis zum Kontaktpunkt P[9],
- vom Kontaktpunkt P[9] bis zum Kontaktpunkt P[10],
- vom Kontaktpunkt P[10] bis zum Kontaktpunkt P[17],
- vom Kontaktpunkt P[17] bis zum Kontaktpunkt P[18],
- vom Kontaktpunkt P[18] bis zum Kontaktpunkt P[4],
- vom Kontaktpunkt P[4] bis zum Kontaktpunkt P[3] und
- vom Kontaktpunkt P[3] im Schussfaden S[6] bis in die Anschlusslasche 120.

Im vorliegenden Ausführungsbeispiel ist der Kettfaden K[3] ein hochohmiger Faden (im Ausführungsbeispiel 10⁵ Ohm/cm), während alle anderen elektrisch leitfähigen Fäden niederohmig sind. Durch die Verknüpfung des Kettfadens K[3] mit dem Schussfaden S[2] am Kontaktpunkt P[5] und die Verknüpfung des Kettfadens K[3] mit dem Schussfaden S[10] am Kontaktpunkt P[6] wird zwischen den Kontaktpunkten P[5] und P[6] ein hochohmiger Abschlusswiderstand mit genau definierter Länge und innerhalb vorgegebener Toleranzen spezifiziertem Widerstandswert als Abschnitt der Leiterbahnen erzeugt. Der so erzeugte Abschlusswiderstand weist eine hinreichende Reproduzierbarkeit in der Massenfertigung auf und dient als Kontrollabschnitt zur Funktionsüberprüfung des Feuchtigkeitssensors im trockenen Zustand.

Wenn der Feuchtigkeitssensor 100 an eine elektrische Auswerteeinheit (nicht gezeigt) angeschlossen wird und eine im Wesentlichen konstante Prüfspannung zwischen den freien Enden der Schussfäden S[5] und S[6] auf der Anschlusslasche angelegt wird, so fließt ein elektrischer Strom durch die gesamte Leiterbahn.

Im trockenen Zustand des Feuchtigkeitssensors entspricht der gemessene Ohm'sche Widerstand der Leiterbahn im Wesentlichen dem vorbekannten Ohm'schen Widerstand des Abschlusswiderstands. Bei einem Leiterbahnbruch oder einer mangelhaften oder fehlenden Kontaktierung der Anschlussklemme (nicht gezeigt) mit den Schussfäden in der Anschlusslasche ist der gemessene Ohm'sche Widerstand der Leiterbahn wesentlich höher als der vorbekannte Ohm'sche Widerstand des Abschlusswiderstands, z.B. unendlich hoch. Bei einem feuchten Feuchtigkeitssensor ist der gemessene Ohm'sche Widerstand wesentlich kleiner als der vorbekannte Ohm'sche Widerstand des Abschlusswiderstands. Der gemessene Ohm'sche Widerstand kann in der Auswerteeinheit mit einem unteren Grenzwert und einem oberen Grenzwert verglichen werden. Wenn der gemessene Ohm'sche Widerstand den oberen Grenzwert überschreitet, so wird auf einen defekten Sensor oder auf einen fehlerhaften Anschluss des Feuchtigkeitssensors an der Auswerteeinheit geschlossen. Wenn der gemessene Ohm'sche Widerstand den unteren Grenzwert unterschreitet, so wird von der Auswerteinheit Feuchtigkeit erkannt.

Es ist analog dazu natürlich auch möglich, den elektrischen Strom durch die Leiterbahn zu messen und auszuwerten. Der gemessene elektrische Strom kann in der Auswerteeinheit mit einem unteren Grenzwert und einem oberen Grenzwert verglichen werden. Wenn der gemessene elektrische Strom den oberen Grenzwert überschreitet, so wird von der Auswerteinheit Feuchtigkeit erkannt. Wenn der gemessene elektrische Strom den unteren Grenzwert unterschreitet, so wird auf einen defekten Sensor oder auf einen fehlerhaften Anschluss des Feuchtigkeitssensors an der Auswerteeinheit geschlossen.

Beim Anschließen des trockenen Feuchtigkeitssensors an die Auswerteeinheit wird eine Basismessung des Ohm'schen Widerstands und/oder des elektrischen Strom zur Kalibrierung des Auswerteeinheit durchgeführt und es werden automatisch die unteren Grenzwerte und die oberen Grenzwerte in Bezug auf die Basismessung festgelegt. Für die Festlegung der unteren Grenzwerte und die oberen Grenzwerte in Bezug auf die Basismessung sind in der Auswerteeinheit Erfahrungswerte von Versuchen mit sehr vielen Feuchtigkeitssensoren des gleichen Typs hinterlegt.

Die begrenzten Abmessungen eines Feuchtigkeitssensors zur Überwachung eines Gefäßzugangs eines Patienten schränken das Platzangebot für einen gewebten Abschlusswiderstand ein. Ein möglichst kurzer Abschlusswiderstand wäre aus diesem Grunde wünschenswert. Hochohmige Fäden weisen jedoch in der Praxis entlang der Fadenlänge Abweichungen der elektrischen Eigenschaften, insbesondere des spezifischen elektrischen Widerstands, von den durchschnittlichen Herstellerangaben auf. Das bedeutet, dass eine gute Reproduzierbarkeit des elektrischen Widerstandswerts eines Abschlusswiderstands eine gewisse wirksam stromdurchflossene Mindestlänge erfordert. Erfahrungen der Anmelderin haben gezeigt, dass eine hohe Reproduzierbarkeit der Widerstandswerte eines so hergestellten Abschlusswiderstands gegeben ist, wenn die stromdurchflossene Länge des hochohmigen Fadens mindestens 10 mm, bevorzugt aber rund 50 mm oder länger beträgt.

Es ist auch möglich, anstelle eines einzelnen Fadens als Abschlusswiderstand einen Bereich von mehreren Fäden als Abschlusswiderstand zu verwenden.
Es ist in alternativen Ausführungsformen auch möglich, die stromdurchflossene Länge des Abschlusswiderstands zur Verbesserung der Reproduzierbarkeit durch konstruktive Maßnahmen zu verlängern.

In einer alternativen Ausführungsform zu der in Figur 1 gezeigten Ausführungsform werden mehrere parallelgeschaltete hochohmige Fäden im Gewebe eingewebt, so dass auf diese Weise die gesamte wirksame stromdurchflossene Länge des hochohmigen Fadens vergrößert wird.

In einer weiteren alternativen Ausführungsform kann eine besonders lange stromdurchflossene Länge des Abschlusswiderstands erzielt werden, wenn ein hochohmiger Faden entlang eines Teils der Außenkontur des Feuchtigkeitssensors angeordnet und an zwei Kontaktstellen der Außenkontur mit den Enden von zwei niederohmigen Leiterbahnen, die nicht mit den niederohmigen Leiterbahnen in der Anschlusslasche zusammenfallen dürfen, kontaktiert wird. Eine solche Anordnung ist allerdings bei einer abgerundeten Außenkontur wie in der Figur 1 gezeigt, nicht alleine durch Weben möglich. Bei einem rechteckigen Feuchtigkeitssensor wäre dies jedoch allein durch Weben möglich. Bei einer solchen Anordnung des Anschlusswiderstands sind die Kreuzungspunkte des hochohmigen Fadens mit den niederohmigen Fäden im mehrlagigen Gewebe als Isolationsstellen auszuführen.

In anderen Ausführungsformen sind weitere konstruktive Merkmale zur Vergrößerung der stromdurchflossene Länge des hochohmigen Fadens vorgesehen. Beispielsweise kann ein aufgebrachtes hochohmiges Firmenlogo Teil des gewebten hochohmigen Abschlusswiderstands sein. Dies ist allerdings nur in bestimmten Ausführungsformen allein durch Weben möglich.

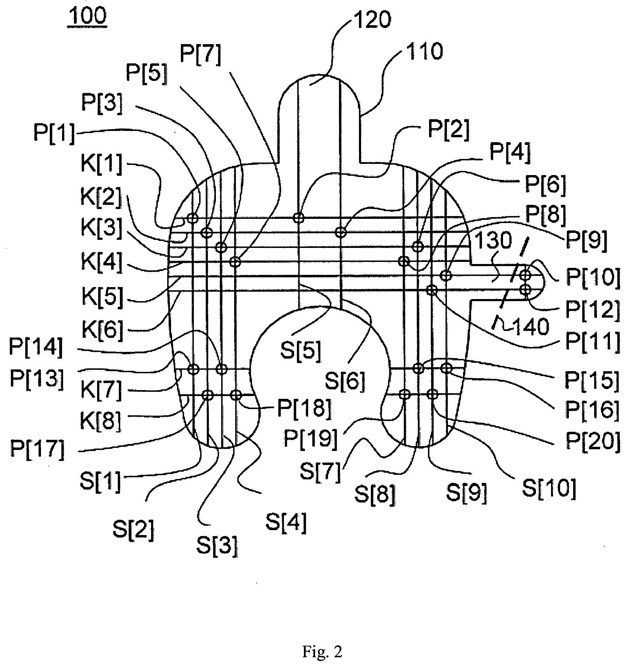

Die **Figur 2** zeigt als zweites Ausführungsbeispiel eine schematische Darstellung eines gewebten Feuchtigkeitssensors mit einer Außenkontur 110 und einer Anschlusslasche 120 und einem durch Weben hergestellten Kontrollabschnitt 130 der Leiterbahn zur Funktionsüberprüfung der Leiterbahn, wobei der Kontrollabschnitt aus einer gewebten Kontrolllasche 130 besteht, die im Wesentlichen über die Kontur des gewebten Feuchtigkeitssensors hinausragt und einen niederohmigen Leiterbahnabschnitt enthält, der Kontaktpunkte P[10] und P[12] enthält.

Zur Funktionsüberprüfung der Leiterbahn kann der Widerstand der gesamten niederohmigen Leiterbahn gemessen werden. Es ist auch möglich, den elektrischen Strom durch die niederohmige Leiterbahn bei der Funktionskontrolle zu messen und mit einem unteren Grenzwert und einem oberen Grenzwert zu vergleichen. Nach Abschluss der Funktionsüberprüfung wird der Sensor sensitiv gemacht, indem die gewebte Kontrolllasche 130 samt den Kontaktpunkten P[10] und P[12] entlang einer in Figur 2 gestrichelt angedeuteten Linie 140 vom gewebten Feuchtigkeitssensor abgeschnitten wird, so dass die geschlossene Leiterbahn in eine erste und eine zweite Elektrode aufgetrennt wird. Die erste Elektrode und die zweite Elektrode sind nach dem Abschneiden der Kontrolllasche 130 voneinander elektrisch isoliert.

Die erste Elektrode erstreckt sich über die miteinander verbundenen Fadenabschnitte:
- Teilabschnitt im Schussfaden S[5] von der Anschlusslasche 120 bis zum Kontaktpunkt P[2]
- vom Kontaktpunkt P[2] bis zum Kontaktpunkt P[1],
- vom Kontaktpunkt P[1] bis zum Kontaktpunkt P[13],
- vom Kontaktpunkt P[13] bis zum Kontaktpunkt P[14],
- vom Kontaktpunkt P[14] bis zum Kontaktpunkt P[5],
- vom Kontaktpunkt P[5] bis zum Kontaktpunkt P[6],
- vom Kontaktpunkt P[6] bis zum Kontaktpunkt P[15],
- vom Kontaktpunkt P[15] bis zum Kontaktpunkt P[16],
- vom Kontaktpunkt P[16] bis zum Kontaktpunkt P[9],
- vom Kontaktpunkt P[9] im Kettfaden K[5] bis zur Schnittlinie 140.

Die zweite Elektrode erstreckt sich über die miteinander verbundenen Fadenabschnitte:
- Teilabschnitt im Schussfaden S[6] von der Anschlusslasche 120 bis zum Kontaktpunkt P[4],
- vom Kontaktpunkt P[4] bis zum Kontaktpunkt P[3],
- vom Kontaktpunkt P[3] bis zum Kontaktpunkt P[17],
- vom Kontaktpunkt P[17] bis zum Kontaktpunkt P[18],
- vom Kontaktpunkt P[18] bis zum Kontaktpunkt P[7],
- vom Kontaktpunkt P[7] bis zum Kontaktpunkt P[8],
- vom Kontaktpunkt P[8] bis zum Kontaktpunkt P[19],
- vom Kontaktpunkt P[19] bis zum Kontaktpunkt P[20],
- vom Kontaktpunkt P[20] bis zum Kontaktpunkt P[11],
- vom Kontaktpunkt P[11] im Kettfaden K[6] bis zur Schnittlinie 140.

Das Abschneiden der Kontrolllasche 130 kann beispielsweise mit einer Schere erfolgen, nachdem der Feuchtigkeitssensor nahe der Punktionsstelle des Gefäßzugangs auf die Haut des Patienten aufgeklebt und hinsichtlich seiner Funktion überprüft wurde. Der Leiterbahnabschnitt zwischen den Kontaktpunkten P[10] und P[12] wurde konstruktiv kurz gewählt, um den Materialverlust gering und die wirksame Elektrodenlänge groß zu halten. Dazu wurden die Kontaktpunkte P[10] und P[12] mit den Kettfäden K[5] und K[6] gebildet, die vorteilhaft nahe beieinander liegen und parallel verlaufen. Somit kann die Kontrolllasche 130 klein ausgeführt werden. Dennoch ist die Kontrolllasche gerade groß genug, um diese beim Abschneiden am Patienten mit sterilen Handschuhen sicher greifen und mit einer Schere abschneiden zu können.

Bei dieser Ausführungsform ist nach dem Abtrennen des Kontrollabschnitts von dem Feuchtigkeitssensor ohne weiteres keine Funktionskontrolle mehr möglich. Belastungstests der Anmelderin an gewebten Feuchtigkeitssensoren haben jedoch gezeigt, dass gewebte Feuchtigkeitssensoren, insbesondere solche ohne hochohmige Fäden, äußerst robust sind gegen die beim praktischen Einsatz in der Klinik auftretenden mechanischen Belastungen. Es ist deshalb ausreichend, den Feuchtigkeitssensor unmittelbar vor der Anwendung hinsichtlich seiner Funktion zu testen und danach auf weitere Funktionstests während der extrakorporalen Blutbehandlung zu verzichten.

Bei dieser Ausführungsform wird kein hochohmiger Abschlusswiderstand benötigt. Dies bedeutet einen Kostenvorteil, weil keine kostenintensiven hochohmigen Fäden benötigt werden.

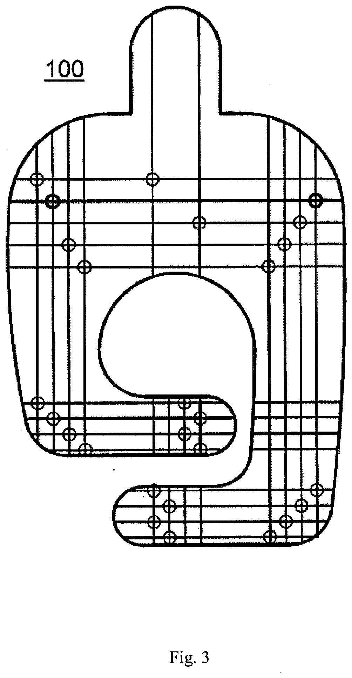

Die **Figur 3** zeigt als drittes Ausführungsbeispiel eine schematische Darstellung eines gewebten Feuchtigkeitssensors 100 ähnlich dem ersten Ausführungsbeispiel aus Figur 1, jedoch mit dem zusätzlichen Merkmal, dass die beiden seitlichen Schenkel des Feuchtigkeitssensors jeweils verlängert ausgeführt sind und jeweils abgewinkelt um die zentrale Ausnehmung des Feuchtigkeitssensors herum geführt sind, wobei die abgewinkelten Bereiche der beiden seitlichen Schenkel beabstandet aneinander vorbeigeführt sind und die zentrale Ausnehmung umschließen, so dass die zentrale Ausnehmung des Feuchtigkeitssensors allseitig von sensitiven Bereichen umgeben ist, ohne dass sich die die abgewinkelten Bereiche der beiden seitlichen Schenkel berühren. Die Lage der Kontaktpunkte des Feuchtigkeitssensors aus Figur 3 ist im Vergleich zur Figur 1 der geänderten Außenkontur des Feuchtigkeitssensors angepasst. Mit dem Feuchtigkeitssensor gemäß Figur 3 kann die Wahrscheinlichkeit weiter verringert werden, dass Flüssigkeit aus dem Bereich der zentralen Ausnehmung heraus zwischen den beiden seitlichen Schenkeln abfließt ohne detektiert zu werden.

Erfindungsgemäß gelingt die Lösung der Aufgabenstellungen der vorliegenden Erfindung mit den gezeigten Ausführungsbeispielen. Die vorliegende Erfindung ist jedoch nicht auf die Ausführungsbeispiele beschränkt.

**Bezugszeichenliste**

| Bezugszeichen | Bezeichnung |
|---|---|
| Feuchtigkeitssensor | 100 |
| Außenkontur des Feuchtigkeitssensors | 110 |
| Anschlusslasche | 120 |
| Kontrolllasche | 130 |
| Schnittlinie | 140 |
| Schussfäden | S[i], i = 1, ..., n |
| Kettfäden | K[i], i = 1, ..., m |
| Kontaktepunkte | P[i], i = 1, ..., k |

## Patentansprüche

1. Gewebter Feuchtigkeitssensor (100) zur Überwachung eines Gefäßzugangs eines Patienten mit mindestens einer durch Weben hergestellten Leiterbahn (S[1] bis S[10]; K[1] bis K[7]), wobei
die Leiterbahn (S[1] bis S[10]; K[1] bis K[7]) einen durch Weben hergestellten Kontrollabschnitt (K[3], P[5], P[6]; 130, P[10], P[11]) aufweist, der zur Funktionsüberprüfung der Leiterbahn (S[1] bis S[10]; K[1] bis K[7]) konfiguriert ist,
der durch Weben hergestellte Kontrollabschnitt (K[3], P[5], P[6]) der Leiterbahn (S[1] bis S[10]; K[1] bis K[7]) als Abschlusswiderstand konfiguriert ist,
der Abschlusswiderstand (K[3], P[5], P[6]) ein hochohmiger elektrisch leitfähiger Faden ist und der spezifische elektrische Widerstand des Kontrollfadens (K[3], P[5], P[6]) wesentlich größer ist als der spezifische elektrische Widerstand der die Leiterbahn ausbildenden elektrisch leitfähigen Fäden, und
der hochohmige elektrisch leitfähige Faden (K[3], P[5], P[6]) einen auf die Länge des Fadens bezogenen spezifischen elektrischen Widerstand im Wertebereich von 10³ Ohm/cm bis 10⁶ Ohm/cm aufweist,
**dadurch gekennzeichnet, dass** der hochohmige elektrisch leitfähige Faden (K[3], P[5], P[6]) eine Carbon-Nanotube-Beschichtung aufweist.

2. Gewebter Feuchtigkeitssensor gemäß Anspruch 1 **dadurch gekennzeichnet, dass** der durch Weben hergestellte Kontrollabschnitt (130, P[10], P[11]) der Leiterbahn (S[1] bis S[10]; K[1] bis K[7]) ein zum Abtrennen von der Leiterbahn vorgesehener und konfigurierter Abschnitt der Leiterbahn ist.

3. Gewebter Feuchtigkeitssensor gemäß Anspruch 2, dass der zum Abtrennen von der Leiterbahn vorgesehene und konfigurierte Kontrollabschnitt (130, P[10], P[11]) der Leiterbahn (S[1] bis S[10]; K[1] bis K[7]) in einem zum zerstörenden Abtrennen vorgesehenen und konfigurierten Bereich des gewebten Feuchtigkeitssensors angeordnet ist.

4. Gewebter gemäß Anspruch 3, **dadurch gekennzeichnet, dass** der zum zerstörenden Abtrennen vorgesehene und konfigurierte Bereich des gewebten Feuchtigkeitssensors (130) ein zum Abschneiden vorgesehener und konfigurierter Bereich des gewebten Feuchtigkeitssensors ist.

5. Gewebter Feuchtigkeitssensor gemäß Anspruch 4, **dadurch gekennzeichnet, dass** der zum Abschneiden vorgesehene und konfigurierte Bereich des Feuchtigkeitssensors eine Lasche (130) ist.

6. Verfahren zur Überwachung eines Gefäßzugangs eines Patienten mit den Schritten Anbringen eines Feuchtigkeitssensors (100) gemäß einem der Ansprüche 3 bis 5 nahe der Punktionsstelle des Gefäßzugangs des Patienten,
Kontaktieren des Feuchtigkeitssensors mit einer Auswertevorrichtung zur Überwachung des Gefäßzugangs eines Patienten, vorgesehen und konfiguriert zur Messung von Feuchtigkeit mit einem Feuchtigkeitssensor (100) gemäß einem der Ansprüche 3 bis 5 durch Messung des elektrischen Widerstands zwischen den Anschlusskontakten des Feuchtigkeitssensors oder durch Messung des elektrischen Stroms in der Leiterbahn des Feuchtigkeitssensors,
Messen des elektrischen Widerstands des Feuchtigkeitssensors (100) oder des elektrischen Stroms in der Leiterbahn, und
Überwachen des Gefäßzugangs des Patienten durch Überwachung des gemessenen elektrischen Widerstands des Feuchtigkeitssensors oder des gemessenen elektrischen Stroms in der Leiterbahn, und mit dem zusätzlichen Schritt des zerstörenden Abtrennens des zum zerstörenden Abtrennen vorgesehenen und konfigurierten Bereichs des gewebten Feuchtigkeitssensors (100) gemäß einem der Ansprüche 3 bis 5 vor dem Beginn der Überwachung eines Gefäßzugangs.

7. Verfahren nach Anspruch 6, **dadurch gekennzeichnet, dass** die Auswertevorrichtung zur Überwachung des Gefäßzugangs eines Patienten, zusätzlich konfiguriert ist zum Vergleichen des gemessenen elektrischen Widerstands mit einem unteren Widerstands-Grenzwert oder einem oberen Widerstands-Grenzwert oder zum Vergleichen des gemessenen Stroms mit einem unteren Strom-Grenzwert oder einem oberen Strom-Grenzwert, wobei auf eine fehlerhafte Leiterbahn geschlossen wird, wenn der gemessene Ohm'sche Widerstand einen ersten unteren Widerstands-Grenzwert unterschreitet oder der gemessene Ohm'sche Widerstand einen ersten oberen Widerstands-Grenzwert überschreitet oder wenn der gemessene Strom einen ersten unteren Strom-Grenzwert unterschreitet oder der gemessene Strom einen ersten oberen Strom-Grenzwert überschreitet.

## Claims

1. A woven moisture sensor (100) for monitoring a patient's vascular access having at least one conductor (S[1] to S[10]; K[1] to K[7]) produced by weaving,
the conductor (S[1] to S[10]; K[1] to K[7]) having a monitoring section (K[3], P[5], P[6]; 130, P[10], P[11]) of the conductor which is produced by weaving, the monitoring section being configured for function-testing the conductor (S[1] to S[10]; K[1] to K[7]),
the monitoring section (K[3], P[5], P[6]) of the conductor (S[1] to S[10]; K[1] to K[7]) which is produced by weaving being configured as a terminating resistor,
the terminating resistor (K[3], P[5], P[6]) being a high-resistance electrically conductive thread and the specific electrical resistivity of the control thread (K[3], P[5], P[6]) being much greater than the specific electrical resistivity of the electrically conductive threads forming the conductor, and
the high-resistance electrically conductive thread (K[3], P[5], P[6]) having a specific electrical resistivity in the value range from 10³ ohm/cm to 10⁶ ohm/cm, based on the length of the thread.
**characterized in that** the high-resistance electrically conductive thread (K[3], P[5], P[6]) has a carbon nanotube coating.

2. The woven moisture sensor according to Claim 1, **characterized in that** the monitoring section (130, P[10], P[11]) of the conductor (S[1] to S[10]; K[1] to K[7]) which is produced by weaving is a section of the conductor which is provided and configured for being cut off from the conductor.

3. The woven moisture sensor according to Claim 2, **characterized in that** the monitoring section (130, P[10], P[11]) of the conductor (S[1] to S[10]; K[1] to K[7]), which is configured and provided for separation from the conductor, is arranged in an area of the woven moisture sensor which is configured and provided for destructive separation.

4. The woven moisture sensor according to Claim 3, **characterized in that** the area of the woven moisture sensor, which is provided and configured for destructive separation, is an area of the woven moisture sensor that is provided and configured for being cut off.

5. The woven moisture sensor according to Claim 4, **characterized in that** the area of the moisture sensor, which is provided and configured for being cut off, is a tongue (130).

6. A method for monitoring a patient's vascular access, having the steps:
applying a moisture sensor (100) according to any one of Claims 3 to 5 close to the puncture site of the patient's vascular access,
contacting the moisture sensor with an evaluation device for monitoring a patient's vascular access which is provided and configured for measuring the moisture with a moisture sensor (100) according to any one of Claims 3 to 5 by measuring the electrical resistance between the terminal contacts of the moisture sensor or for measuring the electrical current in the conductor of the moisture sensor,
measuring the electrical resistance of the moisture sensor (100) or of the electrical current in the conductor, and
monitoring the patient's vascular access by monitoring the measured electrical resistance of the moisture sensor or of the measured electrical current in the conductor, and having the additional step,
destructive separation the area of the woven moisture sensor which is provided and configured for destructive separation according to any ane Claims 3 to 5 before the start of the monitoring of the vascular access.

7. Method according to Claim 6, **characterized in that** the evaluation device for monitoring a patient's vascular access is additionally configured for comparing the measured electrical resistance with a lower resistance limit value or an upper resistance limit value or for comparing the measured current with a lower current limit value or an upper current limit value, a defective conductor being inferred when the measured ohmic resistance falls below a first lower resistance limit value or the measured ohmic resistance exceeds a first upper resistance limit value or when the measured current falls below a first lower current limit value or the measured current exceeds a first upper current limit value.

## Revendications

1. Capteur d'humidité (100) tissé servant à surveiller un accès vasculaire d'un patient avec au moins une piste conductrice (S[1] à S[10] ; K[1] à K[7]) fabriquée par tissage, dans lequel
la piste conductrice (S[1] à S[10] ; K[1] à K[7]) présente une section de contrôle (K[3], P[5], P[6] ; 130, P[10], P[11]) fabriquée par tissage, laquelle est configurée pour surveiller le fonctionnement de la piste conductrice (S[1] à S[10] ; K[1] à K[7]),
la section de contrôle (K[3], P[5], P[6]) fabriquée par tissage de la piste conductrice (S[1] à S[10] ; K[1] à K[7]) est configurée sous la forme d'une résistance de terminaison,
la résistance de terminaison (K[3], P[5], P[6]) est un fil électroconducteur à valeur ohmique élevée et la résistance électrique spécifique du fil de contrôle (K[3], P[5], P[6]) est sensiblement plus importante que la résistance électrique spécifique des fils électroconducteurs réalisant la piste conductrice, et
le fil électroconducteur à valeur ohmique élevée (K[3], P[5], P[6]) présente une résistance électrique spécifique rapportée à la longueur du fil située dans la plage de valeurs de 10³ ohm/cm à 10⁶ ohm/cm,
**caractérisé en ce que** le fil électroconducteur à valeur ohmique élevée (K[3], P[5], P[6]) présente un revêtement de nanotube de carbone.

2. Capteur d'humidité tissé selon la revendication 1, **caractérisé en ce que** la section de contrôle (130, P[10], P(11]) fabriquée par tissage de la piste conductrice (S[1] à S[10] ; K[1] à K[7]) est une section de la piste conductrice prévue et configurée pour être séparée de la piste conductrice.

3. Capteur d'humidité tissé selon la revendication 2, **caractérisé en ce que** la section de contrôle (130, P[10], P[11]), prévue et configurée pour être séparée de la piste conductrice, de la piste conductrice (S[1] à S[10] ; K[1] à K[7]) est disposée dans une zone, prévue et configurée pour être séparée par destruction, du capteur d'humidité tissé.

4. Tissé selon la revendication 3, **caractérisé en ce que** la zone, prévue et configurée pour être séparée par destruction, du capteur d'humidité tissé (130) est une zone, prévue et configurée pour être découpée, du capteur d'humidité tissé.

5. Capteur d'humidité tissé selon la revendication 4, **caractérisé en ce que** la zone, prévue et configurée pour être découpée, du capteur d'humidité est une patte (130).

6. Procédé de surveillance d'un accès vasculaire d'un patient comprenant les étapes de la mise en place d'un capteur d'humidité (100) selon l'une quelconque des revendications 3 à 5 à proximité du point de ponction de l'accès vasculaire du patient,
la mise en contact du capteur d'humidité avec un dispositif d'évaluation servant à surveiller l'accès vasculaire d'un patient, prévu et configuré pour mesurer l'humidité avec un capteur d'humidité (100) selon l'une quelconque des revendications 3 à 5 en mesurant la résistance électrique entre les contacts de raccordement du capteur d'humidité ou en mesurant le courant électrique dans la piste conductrice du capteur d'humidité,
la mesure de la résistance électrique du capteur d'humidité (100) ou du courant électrique dans la piste conductrice, et
la surveillance de l'accès vasculaire du patient en surveillant la résistance électrique mesurée du capteur d'humidité ou du courant électrique mesuré dans la piste conductrice, et comprenant l'étape supplémentaire de la séparation par destruction de la zone, prévue et configurée pour être séparée par destruction, du capteur d'humidité (100) tissé selon l'une quelconque des revendications 3 à 5 avant le début de la surveillance d'un accès vasculaire.

7. Procédé selon la revendication 6, **caractérisé en ce que** le dispositif d'évaluation servant à surveiller l'accès vasculaire d'un patient est configuré en supplément pour comparer la résistance électrique mesurée à une valeur limite de résistance inférieure ou à une valeur limite de résistance supérieure ou pour comparer le courant mesuré à une valeur limite de courant inférieur ou à une valeur limite de courant supérieure, dans lequel on conclut que la piste conductrice est défaillante quand la résistance ohmique mesurée présente une valeur inférieure à une première valeur limite de résistance inférieure ou quand la résistance ohmique mesurée dépasse une première valeur limite de résistance supérieure ou quand le courant mesuré présente une valeur inférieure à une première valeur limite de courant inférieure ou quand le courant mesuré dépasse une première valeur limite de courant supérieure.
